# EUROPEAN PATENT SPECIFICATION

(11) **EP 1 569 663 B1**
(45) Date of publication and mention of the grant of the patent: **28.04.2010**
(21) Application number: 03808347.3
(22) Date of filing: 02.12.2003
(51) Int. Cl.: A61K 31/724, A61K 31/33, A61K 9/08, A61K 47/40, A61K 47/48

(54) **IFOSFAMIDE COMPOSITIONS FOR PARENTERAL ADMINISTRATION AND A PROCESS FOR THEIR PREPARATION**
IFOSFAMID-ZUSAMMENSETZUNGEN ZUR PARENTERALEN VERABREICHUNG UND VERFAHREN FÜR IHRE HERSTELLUNG
COMPOSITIONS A BASE D'IFOSFAMIDE POUR L'ADMINISTRATION PARENTERALE ET LEUR PROCEDE DE PREPARATION

(30) Priority: 02.12.2002 IN MU07852002
(43) Date of publication of application: 07.09.2005
(73) Proprietor: Bharat Serums & Vaccines Ltd., Wagle Estate, Thane 400 604 (IN)
(72) Inventor: DAFTARY, Gautam V., Bharat Serums & Vaccines Ltd., Thane 400 604 (IN); PAI, Srikanth, A., Bharat Serums & Vaccines Ltd., Thane 400 604 (IN); RIVANKAR, S. H., Bharat Serums & Vaccines Ltd., Thane 400 604 (IN); PRAVEEN, Kumar S., Bharat Serums & Vaccines Ltd., Thane 400 604 (IN)
(74) Representative: Duffy, Assumpta Dympna
(86) International application number: PCT/IN2003/000376
(87) International publication number: WO 2004/050012

(56) References cited:
- EP-A- 1 396 268
- WO-A-2004/022699
- WO-A1-02/02125
- US-A- 4 959 215
- US-A- 4 983 586
- US-A- 5 158 779
- US-A- 5 227 373
- US-A- 5 750 131
- US-A1- 2004 186 074
- CHEUNG B.W.Y. ET AL: 'Brain delivery of carbamazepine during intravenous administration of polyethylene glycol and 2-hydroxypropyl-beta-cyclodextrin formulations' REVUE STP PHARMA SCIENCE vol. 7, no. 1, January 1997 - February 1997, pages 78 - 84, XP008035836

## Description

### Field of Invention

This invention relates to Ifosfamide compositions for parenteral use. This invention particularly relates to stable, clear, aqueous Ifosfamide compositions having reduced toxicity, for parenteral administration. This invention more particularly relates Ifosfamide compositions comprising 2-hydroxypropyl-β-cyclodextrin (referred to hereinafter as "HPBCD") in addition to Ifosfamide.

### Background of the invention

Two main groups of drugs used in the treatment of malignant disease are alkalyting agents and the antimetabolites. Ifosfamide is one of the widely used antineoplastic drug belonging to the alkalyting agents group.

Ifosfamide is chemically 3-(2-chtoroethyl)-2-[(2-chloroethyl)amino]-tetrahydro-2H- 1,3,2-oxazaphosphorin-2-oxide and is represented by the formula:

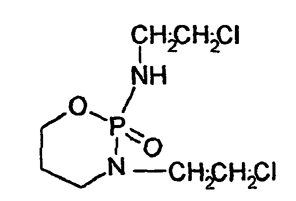

Ifosfamide is a white crystalline hygroscopic powder having a low melting point of 40°C. The powder has a water solubility of about 100mg/ml.

Ifosfamide is used in the treatment of a variety of solid tumours including those of the cervix, endometrium, lung, ovary, testes and thymus as well as in sarcoma and in the treatment of Burkitts lymphoma.

The treatment with Ifosfamide is associated with serious side effects like hemorrhagic cystitis, myelosuppression, cardiac arrythmias, CNS disturbances, nephrotoxicity, haematological and gastro-intestinal reactions. The LD₅₀ in mouse on intravenous administration has been reported to be 338 mg/kg body weight. Combination with the uroprotective agent mesna reduces the incidence of haemorrhagic cystitis. Therefore mesna is normally administered intravenously at a dose of 20% of the Ifosfamide dose at time zero (the time of administration of Ifosfamide), and then at 4 and 8 hours.

Ifosfamide is given intravenously either by injection or by infusion as a diluted solution containing less than 4%w/v of Ifosfamide. Ifosfamide is very susceptible to hydrolytic degradation and accordingly prompt administration of such solutions is generally required. Therefore commercially it is predominately available in dry form and is supplied as sterile packaged dry powder for dissolution in water for injection prior to administration. However, the low melting point and the hygroscopic nature of Ifosfamide make it necessary to fill the powder with great care keeping both temperature and humidity accurately controlled to get a sterile product. Further, prolonged storage of the dry powder also results in sintering and yellowing, which in turn leads to a reduction in dissolution rate thereby increasing time taken for reconstitution.

To overcome difficulties associated with the thermal and hydrolytic susceptibility, lyophilization of the drug has been tried. However, the lyophilization process is quite time consuming requiring specialized equipments. Personnel exposure to the strongly cytotoxic Ifosfamide occurring during reconstitution of lyophilized powder is undesirable.

Hence, attempts are being made to prepare clear liquid Ifosfamide compositions that will be stable over a period of time for parenteral administration.

US 4952575 discloses preparation of ethanolic solution of Ifosfamide containing 96% to 100% ethanol. Even though the degradation of Ifosfamide has been shown to be minimal, use of solvents in such a high concentration leads to other problems such as volatility, handling during manufacturing, miscibility with blood on administration. As such alcohol is pharmacologically active which may also affect the person on administration of alcoholic solution of Ifosfamide.

There are two patents using polyols for making stable liquid compositions of oxazophosphorine compounds namely

US 4879286 discloses an invention in which Cyclophosphamide is formulated in a ready-to-dilute solution. This invention uses organic polyols namely propylene glycol and polyethylene glycol and their mixtures as a solvent and also 0 to 50% water. The water may be partly replaced by 10 to 30% of ethanol.

WO 0202125 discloses liquid pharmaceutical composition for parenteral administration comprising Ifosfamide, solvent and optionally, conventional pharmaceutical carriers and excipients. According to the invention, the solvent comprises 35-75% lower alcohol and 25-65% polyol. While lower alcohol solvent is usually ethanol, the polyol solvent is propylene glycol, glycerol and /or polyethylene glycol.

In both the patents US 4879286 and WO 0202125, the parenteral administration of larger amounts of polyols and alcohols lead to other problems like pain or irritation on injection, hemolysis, ototoxicity, cardiovascular effects, CNS effects and seizures. It may also lead to hyperosmolarity and lactic acidosis in patients with renal impairment.

WO 9918973 describes a stable, ready-to-use liquor of Ifosfamide using Sodium chloride as a stabilizing agent. The invention also discloses 10-500 mg/ml Ifosfamide composition containing Urea, sodium chloride and sodium dihydrogen phosphate. The compositions of the invention are said to be stable but there is no mention about the safety and toxicity of the composition. The higher concentration of urea in the formulation may lead to complications like hemolysis, irritation, phlebitis & thrombosis at the site of injection, and elevated blood ammonia & urea concentrations in patients with hepatic and renal function impairment.

WO 03/051297A2 describes a ready-to-use aqueous composition of Ifosfamide comprising 40 - 400 mM (10 - 100mg/ml) of Ifosfamide in pharmaceutically acceptable buffer. The patent suggests the use of buffers preferably from the group of Na₂HPO₄ & NaH₂PO₄ and K₂HPO₄ & KH₂PO₄. There is no report on the toxicity of Ifosfamide compositions disclosed in this patent.

Thus, there remains a need for a stable, concentrated Ifosfamide solution to facilitate handling during administration. In addition, there remains a need for Ifosfamide pharmaceutical compositions that exhibit less toxicity than the currently available compositions.

Our main object of the present invention is thus to develop stable, clear, aqueous Ifosfamide compositions having reduced toxicity for parenteral administration in human beings and other mammals.

Another object of the invention is to develop a process for making such compositions.

### Summary of the Invention

Accordingly, the present invention provides stable, clear, aqueous Ifosfamide compositions having reduced toxicity, for parenteral administration comprising
Ifosfamide up to 1100 mg /ml of the composition
and 2-hydroxypropyl-β-cyclodextrin, with the proviso that the composition does not contain sodium 2-mercaptoethane sulphonate.

The present invention also provides a process for the preparation of Ifosfamide compositions having reduced toxicity, for parenteral administration comprising steps of
i. bringing in intimate contact Ifosfamide, 2-hydroxypropyl-β-cyclodextrin and water;
ii. rendering sterile the composition so obtained at the end of step (i).

In the process of the present invention, required quantities of pharmaceutically acceptable additives may be added while bringing in intimate contact Ifosfamide, 2-hydroxypropyl-β-cyclodextrin and water.

Intimate contact may be brought about by methods such as stirring, mixing, sonicating, heating, homogenizing. Preferably it is carried out under aseptic conditions and under inert gas atmosphere at temperatures below 40°C.

Further the invention provides adding one or more pharmaceutically acceptable buffers, tonicity agents, preservatives, chelating agents, antioxidants, anticrystallising agents.

The present invention also contemplates rendering the composition sterile preferably by passing through sterile 0.2µm sterilizing grade filter and transferring aseptically the sterile composition into sterile containers followed by purging the air in the headspace of the container with inert gas such as nitrogen and sealing the containers.

### Detailed description of embodiments of the invention

Ifosfamide content in the composition described in this invention is up to 1100 mg/ml., the preferred range is 1 mg/ml to 200 mg/ml, more preferably 10 mg/ml to 100 mg/ml, most preferably 40mg/ml to 50mg/ml. Other preferred ranges of Ifosfamide content in the composition described in this invention are 200mg/ml to 500 mg/ml and 500 mg/ml to 1000 mg/ml.

In concentrated Ifosfamide compositions, Ifosfamide content is from 200 mg/ml to 1100 mg/ml, preferably, 200 mg/ml to 1000 mg/ml, more preferably 500mg/ml and 1000mg/ml.

2-hydroxypropyl-β-cyclodextrin (HPBCD) is a partially substituted poly(hydroxypropyl)ether of beta cyclodextrin. The hydroxyproplyl groups are randomly substituted onto hydroxyl groups of the cyclodextrin and the amount of substitution is reported as average degree of substitution or number of hydroxypropyl groups per cyclodextrin. Alternatively amount of substitution is reported as molar substitution (MS) or the average number of substitution per anhydroglucose unit in the ring of the cyclodextrin. Molar substitution can have an effect on the binding of guest molecules to HPBCD . At low degree of substitution, binding is very similar to that of the unmodified betacyclodextrin. Increasing molar substitution can lead to weakened binding due to stearic hindrance. HPBCD having molar substitution between 0.05 to about 2 can be useful in this invention. HPBCD having molar substitution between 0.3 to about 1.5 is preferred, HPBCD having molar substitution between 0.5 to about 1.2 is more preferred.

HPBCD may be present in the composition at a molar ratio of Ifosfamide to HPBCD 100 : 0.1 to 1 : 300, preferably 100 : 0.25 to 1 : 100, more preferably 100 : 1 to 1 : 20. Other preferred molar ratio of Ifosfamide to HPBCD is 100 : 3.3 to 1 : 2.5.

While making concentrated Ifosfamide compositions the requirement of HPBCD varies with the concentration of Ifosfamide. Initially it increases with the concentration of Ifosfamide. But at the higher end of the Ifosfamide concentration, HPBCD and water content is limited due to volume constraint. Therefore the ratio of Ifosfamide to HPBCD should be rightly chosen.

The pH of the composition of the present invention may be between 3.0 - 9.0. The preferable pH for the present invention is between 5.0 - 8.0.

The compositions of the present invention may also include pharmaceutically acceptable additives for the purposes of pH stabilization, preservation, isotonicity adjustment, stabilization against oxidation, chelating agents, anticrystallising agents and other suitable additives. Some of the pharmaceutically acceptable additives may be present in the aqueous solution to which the Ifosfamide and HPBCD are added and / or some of them may be added separately as a solution in water before making up the volume in the final composition.

The compositions of the present invention may require suitable buffers to adjust or stabilise the pH. Suitable buffering agents for the compositions of the present invention include but are not limited to Phosphate buffer, Citrate buffer, Glycine buffer, Histidine buffer containing any of the commonly used compounds or a mixture of compounds such as Sodium dihydrogen phosphate, Disodium hydrogen phosphate, Potassium dihydrogen phosphate, Dipotassium hydrogen phosphate, Histidine hydrochloride, Sodium hydroxide, Phosphoric acid, Sodium citrate, Citric acid, Glycine, Potassium citrate, Hydrochloric acid and Potassium hydroxide. Preferred buffering agent is a mixture of Sodium dihydrogen phosphate and Disodium hydrogen phosphate.

Of the other pharmaceutically acceptable additives, suitable tonicity agent for compositions of the present invention are selected from group of compounds such as glycerin, sodium chloride, maltose, mannitol, dextrose and mixtures thereof.

Similarly, suitable preservatives for compositions of the present invention may include methyl hydroxy benzoic acid, propyl hydroxy benzoic acid, phenol, benzyl alcohol and sodium benzoate.

The composition of the present invention may contain suitable chelating agents such as ethylenediaminetetraacetic acid (EDTA) & its salts and Desferoximine methane sulfonate (Desferal).

The composition of the present invention may also contain suitable antioxidants such as, ascorbic acid, sodium bisulfite, sodium metabisulfite, butylated hydroxy anisole and butylated hydroxy toluene.

Also, the composition of the present may contain substances such as Glycerin as anticrystallising agents.

The aqueous solutions containing Ifosfamide and HPBCD are brought in intimate contact by methods such as stirring, mixing, sonicating, heating, homogenizing. Pharmaceutically acceptable additives such as buffers, tonicity agents, preservatives, chelating agents, antioxidants, anticrystallising agents as required by parenteral dosage form may be present in the aqueous solution to which the Ifosfamide and HPBCD is added. Alternatively, they can be added separately as a solution in water before making up the volume. In the preparation of concentrated Ifosfamide composition, to incorporate larger amounts of Ifosfamide, HPBCD is dissolved in minimum quantity of water and Ifosfamide is solubilized by intimate stirring. Pharmaceutically acceptable additives if required are added as such or as solutions into Ifosfamide - HPBCD solution. Finally the remaining quantity of water is added to makeup to the required volume, followed by mixing to get a homogenous solution and then rendering sterile the composition so obtained. The composition may be rendered non-pyrogenic if required by passing through Tangential Flow Filtration System (TFF) before sterilisation.

The composition is usually rendered sterile by passing through a sterilising grade filter. Preferably, 0.2µm sterilizing grade filters may be used.

The sterile compositions of the present invention may be filled aseptically into sterile containers such as vials, ampoules, plastic containers, purging the air in the headspace of the containers with inert gas such as nitrogen and sealing the filled containers.

Not bound by theory, we believe that the three component system of Ifosfamide, HPBCD and water is a stable aqueous composition. Such systems are being studied at present for different aspects such as solubility, complexation and encapsulation over different temperature and time period. Further the effect of other additives on the stability of the system is also under study.

It is well known that the treatment with Ifosfamide is associated with serious side effect like hemorrhagic cystitis, and to counteract it Mesna is always administered with Ifosfamide injection. However, there are other side effects like myelosuppression, cardiac arrythmias, Central Nervous System (CNS) disturbances, nephrotoxicity, haematological and gastro-intestinal reactions which are not taken care of by the Ifosfamide compositions available as of today.

The toxicity of the compositions of the present invention were evaluated against the conventional dosage form available in the market (Holoxan). The compositions of present invention when studied in swiss albino mice showed lesser toxic effects like convulsions, myelosuppressions, hepatotoxicity. The mortality rate was found to be significantly less in animals treated with the compositions of the present invention compared to conventional marketed product. LD₅₀ values of the compositions studied are represented in examples.

Not bound by theory, we believe that the Ifosfamide whole or a portion of it is complexed in HPBCD cavity in the aqueous solution. On systemic administration, the drug is released from the cavity in to the blood stream. The free drug and the drug-HPBCD complex will be in equilibrium and the metabolism of the free Ifosfamide shifts the equilibrium resulting in the release of free drug. Presence of Ifosfamide in complex form and optimum levels of free drug in the blood may prevent the interaction of Ifosfamide with healthy tissues and organs thereby preventing undesirable side effects.

In the present invention, Ifosfamide and HPBCD may form a synergistic combination to give reduced toxicity both in presence and absence of Mesna as shown by LD₅₀ values in mice and hemorrhagic cystitis studies in rats. The Ifosfamide compositions of the present invention having reduced toxicity are of advantage in the treatment of a variety of solid tumours including those of the cervix, endometrium, lung, ovary, testes and thymus as well as in sarcoma and in the treatment of Burkitts lymphoma.

The solubility of Ifosfamide in water is about 100mg/ml. The process of the present invention makes it possible to obtain a composition containing Ifosfamide in a concentration greater than 100mg/ml. These concentrated solutions containing 100mg/ml to 1100mg/ml offer additional advantages like safety by virtue of less handling, thereby less exposure of clinicians to cytotoxic Ifosfamide during administration and increased assurance of sterility.

### Examples :

The invention will now be illustrated by way of Examples. The Examples are by way of illustration only.

Ifosfamide used in these Examples was of parenteral grade complying with US Pharmacopoeial specifications. 2-hydroxypropyl-β-cyclodextrin (HPBCD) used was manufactured by Wacker Chemie having molar substitution per anhydroglucose unit by hydroxy propyl groups between 0.5 to 1.2. Equipments used were of conventional nature; the entire processing was done in an area with a controlled environment. Water used in these Examples was of parenteral grade complying with "Water for Injection" specifications. All other additives used in these Examples were of parenteral grade.

### Examples I : Preparation of Ifosfamide 50mg/ml composition containing 20% HPBCD in water

The following composition was prepared by the procedure given below

| | | |
|---|---|---|
| i. | Ifosfamide | 10gm. |
| i. | HPBCD | 40gm |
| ii. | Water | qs to 200ml. |

Weighed quantity of HPBCD was dissolved in 150ml of water. Weighed quantity of Ifosfamide was added and mixed for 3 hours. The volume was made up to 200ml with water and mixed. The resultant solution was filtered through a 0.2µ filter and filled aseptically in sterile glass vials. The glass vials were closed under aseptic conditions with sterile Teflon™ coated rubber bungs and sealed using flip off seals.

The composition obtained in this Example was analyzed for Ifosfamide content by High Pressure Liquid Chromatography (HPLC) method and was found to contain 51.73mg/ml of Ifosfamide. The composition had a pH of 6.5.

### Example II: Stability of composition of Example I

The composition obtained in Example I was subjected to long term Stability studies at 2-8°C. The stability data at the end of 24 months is shown in Table 1.

**Table 1 : Stability data of Composition of Example I**

| **Ifosfamide mg/ml** | **HPBCD %** | **Buffer** | **Initial % Ifosfamide content** | **24 month at 2-8°C** |
|---|---|---|---|---|
| | | | | **% Ifosfamide content** |
| 50 | 20% | --- | 103.46 | 99.12 |

The above data shows insignificant drop in Ifosfamide content indicating a good stability.

### Example III: Toxicity study of composition of Example I

The composition obtained in Example I was subjected to acute toxicity studies in mice

Experimental details are as follows:

| | |
|---|---|
| Animals used | : Swiss albino mice of either sex. |
| Weight range of animals | : 20-22 gm. |
| Number of groups | : 10 |
| Number of animals per group : | 10 |
| Acclimatization | : One week under test conditions under controlled temperature and humidity. |
| | |

| **Test Materials** | : ***Ifosfamide Injection*** |
|---|---|
| Identity | : Composition of Example I |
| Description | : Clear colourless solution |
| Route of administration | : Intravenous |
| | |

| **Comparative material** | :***Holoxan*^{™} (*reconstituted)*** |
|---|---|
| Identity | :Ifosfamide injection U.S.P. |
| Lot No. | : G 220 |
| Manufacturing Date | :October 2001 |
| Expiry Date | : September 2003 |
| Description | :Dry powder for reconstitution with water for injection |
| Strength | :40 mg/ml on reconstitution |
| Manufacturer | : German Remedies Limited. |
| Route of administration | : Intravenous |

Both the drug solutions were suitably diluted with 5% Dextrose Injection and administered intravenously. Ifosfamide in the doses of 400 mg/kg, 500 mg/kg, 600 mg/kg, 700 mg/kg and 800 mg/kg body weight was administered in 10 different groups of animals, each group consisting of 10 animals.

The animals were kept under observation for 14 days and mortality recorded at the end of 7 days. The LD₅₀ dose i.e. the dose that is lethal to 50% of animals is shown in Table 2.

**Table 2.**

| **LD₅₀ dose of Composition of Example I and Holoxan^{™}** | |
|---|---|
| **Composition** | **LD₅₀ (mg/kg Body weight)** |
| Example I | 648.04 |
| Holoxan^{™} | 562.16 |

The above data clearly indicates that composition of Example I is less toxic compared to the Conventional formulation.

### Example IV: Preparation of Ifosfamide composition containing 10% HPBCD in phosphate buffer

The following composition was prepared by the procedure given below

| | | | |
|---|---|---|---|
| i. | Ifosfamide | - | 10g |
| ii. | HPBCD | - | 20g |
| iii. | Disodium hydrogen phosphate | - | 0.1g |
| iv. | Sodium dihydrogen phosphate | - | 0.06g |
| v. | Water | - | q.s. to 200ml |

Weighed quantities of Disodium hydrogen phosphate and Sodium dihydrogen phosphate were dissolved in 160ml of water. Weighed quantity of HPBCD was added and dissolved slowly under stirring in this buffer solution. Weighed quantity of Ifosfamide was gradually added under stirring to the buffered HPBCD solution and mixed for 3 hours. The volume was made up to 200ml with water and mixed. The resultant solution was filtered through a 0.2µ filter and filled aseptically in sterile glass vials. The air in the headspace of the vials was purged with nitrogen and the glass vials were closed under aseptic conditions with sterile Teflon™ coated rubber bungs and sealed using flip off seals.

The composition obtained in this Example was analysed for Ifosfamide content by High Pressure Liquid Chromatography (HPLC) method and was found to contain 50.23mg/ml of Ifosfamide. The composition had a pH of 7.2.

### Comparative Example V : Preparation of Ifosfamide 50mg/ml composition in phosphate buffer

To ascertain the toxicity reducing nature of HPBCD by toxicity studies a comparative composition containing Ifosfamide in buffer was prepared by the procedure given below

| | | | |
|---|---|---|---|
| i. | Ifosfamide | - | 10g |
| ii. | Disodium hydrogen phosphate | - | 0.1g |
| iii. | Sodium dihydrogen phosphate | - | 0.06g |
| iv. | Water | - | q.s. to 200ml |

Weighed quantities of Disodium hydrogen phosphate and Sodium dihydrogen phosphate were dissolved in 180ml of water. Weighed quantity of Ifosfamide was gradually added under stirring to the buffer solution and mixed for 3 hours. The volume was made up to 200ml with water and mixed. The resultant solution was filtered through a 0.2µ filter and filled aseptically in sterile 10ml glass vials. The air in the headspace of the vials was purged with nitrogen gas and the glass vials were closed under aseptic conditions with sterile Teflon™ coated rubber bungs and sealed using flip off seals.

The composition obtained in this Example was analysed for Ifosfamide content by High Pressure Liquid Chromatography (HPLC) method and was found to contain 50.1mg/ml of Ifosfamide. The composition had a pH of 6.5

### Example VI: Acute toxicity study of Example IV and Example V

Experimental details are as follows:

| | |
|---|---|
| Animals used | : Swiss albino mice of either sex. |
| Weight range of animals | : 20-22 gm. |
| Number of groups | : 25 |
| Number of animals per sub group | : 8 |
| Acclimatization | : One week under test conditions under controlled temperature and humidity. |
| | |

| **Test Materials** | :***Ifosfamide Injection*** |
|---|---|
| Identity | : Composition of Example IV |
| Description | : Clear colourless solution |
| Route of administration | : Intravenous |
| | |

| **Comparative material 1.** | : ***Ifosfamide Injection*** |
|---|---|
| Identity | : Composition of Example V |
| Description | : Clear colourless solution |
| Route of administration | : Intravenous |
| | |

| **Comparative material 2.** | ***Holoxan*^{™} *(reconstituted)*** |
|---|---|
| (Details of ***Holoxan*^{™}** are | shown in Example III |
| | |

| **Uroprotective material** | **: *Uromitexan*^{™}** |
|---|---|
| Identity | :Mesna Injection |
| Lot No. | :G 168 |
| Manufacturing Date | :October 2001 |
| Expiry Date | :September 2004 |
| Description | :Clear & colorless solution for Intravenous Injection |
| Strength | :100 mg/ml |
| Manufacturer | : German Remedies Limited. |
| Route of administration | : Intravenous |

The compositions obtained in Example IV and Example V were subjected to acute toxicity studies in mice. A conventional formulation, Holoxan^{™} was reconstituted as directed by the manufacturer and was used as a control. The doses of Ifosfamide selected for the study were 400 mg/kg, 500 mg/kg, 600 mg/kg, 700mg/kg and 800mg/kg body weight. The Ifosfamide solutions of compositions of Example IV, Example V and Holoxan^{™} were suitably diluted with 5% Dextrose Injection as such and also with Mesna at a dose of 20% of Ifosfamide dose and administered intravenously in 25 different groups of animals, each group consisting of eight animals.

The animals were kept under observation for 14 days and mortality recorded at the end of 7 days. The LD₅₀ values of compositions of Example IV, Example V and Holoxan^{™} are shown in Table no 3.

**Table No 3 : The LD₅₀ values of compositions of Example IV, V and Holoxan^{™}**

| **Composition** | **HPBCD concentration** | **Buffer** | **Mesna Dose as % of Ifosfamide Dose** | **LD₅₀ (mg/kg Body weight)** |
|---|---|---|---|---|
| Examples IV | 10% | phosphate | ---- | 661.57 |
| Holoxan^{™} | Nil | nil | ----- | 562.16 |
| Example V | nil | phosphate | ----- | 566.00 |
| **Example IV** | **10%** | **phosphate** | **20** | **669.54** |
| **Holoxan^{™}** | **nil** | **nil** | **20** | **580.00** |

The above data clearly indicates that composition of Example IV is less toxic both in presence and absence of Mesna compared to the Conventional formulation as well as Ifosfamide in buffered solution.

### Example VII: Repeat dose toxicity studies of composition of Example IV

The composition obtained in Example IV along with conventional formulation Holoxan^{™} were subjected to repeat dose toxicity study in mice to evaluate the effect of Ifosfamide compositions on haematological and biochemical parameters.

Experimental details are as follows:

| | |
|---|---|
| Animals used | : Swiss albino mice of either sex. |
| Weight range of animals | : 20-22 gm. |
| Number of groups | : 7 |
| Number of animals per group : | 10 |
| Acclimatization | : One week under test conditions under controlled temperature and humidity. |
| **Test Materials** | : Composition of Example IV |
| **Comparative material.** | **:*Holoxan*^{™} *(reconstituted)*** |
| **Uroprotective material** | **: *Uromitexan*^{™}** |
| ( The details of ***Holoxan*^{™}** and ***Uromitexan*^{™}** are given in Example VI) | |

The animals were injected Ifosfamide along with Mesna (as 20% of Ifosfamide Dose) at daily doses of 80 mg/kg, 100 mg/kg and 120 mg/kg, intravenously for seven days. The untreated group was used as a control. The animals were observed during the study period of 14 days for mortality, haematological and biochemical changes. The total WBC count was done before and after treatment with composition of Example IV and Holoxan^{™}. The values are shown in Table no 4

**Table no 4 : The total WBC count of animals treated with composition of Example IV and Holoxan^{™}.**

| **Dose** | | **Total WBC (cells/micrelitre) ± SEM** | | | | | |
|---|---|---|---|---|---|---|---|
| **(mg/kg body weight)** | | **Examples IV** | | **Holoxan ^{™}** | | **Control** | |
| **Ifosfamide** | **Mesna** | Pre treatment | Post treatment | Pre treatment | Post treatment | Pre treatment | Post treatment |
| 80 | 16 | 6220.00 ± 631.73 | 3170.00 ± 478.21 | 4870.00 ± 310.94 | 1840.00 ± 131.83 | | |
| 100 | 20 | 5760.00 ± 435.44 | 6440.00 ± 1764.2 | 5980.00 ± 806.77 | 2220.00 ± 237.02 | 7920.00 | 10980.0 |
| 120 | 24 | 5900.00 ± 481.91 | 4420.00 ± 1102,0 | 8150.00 ± 904.61 | 1362.50 ± 301.15 | ± 539.93 | ± 666.65 |

The animals treated with Example IV and Holoxan ^{™} showed reduction in total WBC count. However, animals treated with Holoxan ^{™} showed severe leucopoenia compared to composition of Example IV. This indicates less toxic nature of composition of Example IV.

To study the effects of composition of Example IV and Holoxan ^{™} on Liver function, the Serum Glutamate Oxaloacetate Transaminase (SGOT) levels of treated and untreated control group were analysed. The SGOT levels of treated and untreated control groups are shown in Table no 5.

**Table 5 : The average SGOT levels of animals treated with composition of Example IV, Holoxan^{™} and untreated control.**

| Dose of Ifosfamide | Dose of Mesna | SGOT level (U/L) | | |
|---|---|---|---|---|
| (mg/kg body weight) | (mg/kg body weight) | Example IV | Holoxan^{™} | Control |
| 80 | 16 | **129.15** | **158.50** | |
| 100 | 20 | **147.8** | **299.65** | **136.4** |
| 120 | 24 | **164.25** | **309.15** | |

As per the above data the elevation of SGOT levels in animals treated with Example IV was slightly higher than that of control group, whereas in animals treated with Holoxan^{™} increase in SGOT values was highly significant. This indicates reduced hepatotoxic nature of composition of Example IV.

### Example VIII : Hemorrhagic cystitis studies of composition IV

The composition obtained in Example IV along with conventional formulation Holoxan^{™} were subjected to Hemorrhagic cystitis studies in rats to evaluate their bladder toxicity.

Experimental details are as follows:

| | |
|---|---|
| Animals used | : Wistar rats of either sex. |
| Weight range of animals | : 100-150 gm. |
| Number of groups | : 9 |
| Number of animals per group | : 2 |
| Acclimatization | : One week under test conditions under controlled temperature and humidity. |
| | |
| **Test Materials** | **: Composition of Example IV** |
| **Comparative material .** | **: *Holoxan*^{™} *(reconstituted)*** |
| **Uroprotective material** | **: *Uromitexan*^{™}** |
| ( The details of *Holoxan*^{™} and *Uromitexan*^{™} are given in Example VI) | |

### Study design

Animals were divided into 9 groups and each group comprised two animals. Ifosfamide alone and with Mesna was administered via the intravenous route at doses of 400mg/kg and 500mg/kg bodyweight. The group treated with Dextrose Injection was used as a control.

The animals were sacrificed 24 hours after injection. The urinary bladders of all the animals were collected and were fixed in 10% formalin for 48 hours. Histopathological slides of the organ were prepared and subjected to microscopic examination.

### Observations

Table 6 depicts the evaluation results on hemorrhagic cystitis of two formulations of Ifosfamide

**Table 6 : Scoring of hemorrhagic cystitis**

| **Sl no** | **Ifosfamide Dose** | **Mesna Dose** | **Score** | |
|---|---|---|---|---|
| | | | **Example IV** | **Holoxan^{™}** |
| 1 | 400 | - | 1+ | 2+ |
| 2 | 400 | - | N | 3+ |
| 3 | 500 | - | 1+ | 3+ |
| 4 | 500 | - | 1+ | 1+ |
| 5 | 400 | 80 | N | 1+ |
| 6 | 400 | 80 | N | 1+ |
| 7 | 500 | 100 | N | 1+ |
| 8 | 500 | 100 | N | 2+ |

| | | | | |
|---|---|---|---|---|
| **N** : Normal **1 +** : Mild hemorrhagic cystitis **2 +** : Moderate hemorrhagic cystitis with or without epithelial atypia **3 +** : Severe hemorragic cystitis with or without epithelial atypia | | | | |

### Results:

| | |
|---|---|
| Holoxan | : **moderate to severe** hemorrhagic cystitis |
| Holoxan with Mesna | : **mild to moderate** hemorrhagic cystitis |
| Example IV | : **mild to moderate** hemorrhagic cystitis |
| Example IV with Mesna | : **No** hemorrhagic cystitis |

### Conclusion:

The above findings conclusively proved that the composition of Example IV has less bladder toxicity than the conventional formulation Holoxan^{™}

### Example IX: Stability study of composition of Example IV

The composition obtained in Example IV was subjected to stability studies at 2°C -8°C. The samples at the end of 6 and 12 months were analysed by HPLC method. The data is shown in Table no 7

**Table 7: Stability data of composition of Example IV.**

| **Storage condition** | **Description** | **% Ifosfamide content** |
|---|---|---|
| Initial | Clear, colourless liquid | 50.2 mg/ml |
| 2°C-8°C - 6 Months | Clear, colourless liquid | 50.9 mg/ml |
| 2°C-8°C -12 Months | Clear, colourless liquid | 49.1 mg/ml |

The above data shows insignificant drop in Ifosfamide content at 2°C - 8°C indicating good stability over a period of 12 months

The other compositions of the present invention comprising different concentrations of Ifosfamide and HPBCD are shown in the Table no 8

**Table: 8 : Other compositions of the present invention**

| **Ingredients** | **Example** | | | | | | |
|---|---|---|---|---|---|---|---|
| | **X** | **XI** | **XII** | **XIII** | **XIV** | **XV** | **XVI** |
| Ifosfamide | 10.0g | 10.0g | 10.0g | 20.0g | 100g | 100g | 200g |
| HPBCD | 40.0g | 80.0g | 20.0g | 40.0g | 40.0g | 80.0g | 10.0g |
| Disodium hydrogen phosphate | 0.1g | 0.1g | - | 0.1g | - | - | - |
| Sodium dihydrogen phosphate | 0.06g | 0.06g | - | 0.06g | - | - | - |
| Water to make up the volume | qs to 200 ml | qs to 200 ml | qs to 200 ml | qs to 200 ml | qs to 200 ml | qs to 200 ml | qs to 200 ml |
| Ifosfamide concentration | 50mg/ml | 50mg/ml | 50mg/ml | 100mg/ ml | 500mg/ ml | 500mg/ ml | 1000mg /ml |

The compositions shown in table no 8 were prepared by the procedure given below

### Example X :

Ifosfamide (10g) and HPBCD (40g), Disodium hydrogen phosphate (0.1 g) and Sodium hydrogenphosphate (0.06g) were taken in a graduated flask. Water for injection was slowly added into it with intermittent mixing to get 200ml clear homogenous solution. The resultant solution was passed through sterile 0.2µm filter and filled aseptically in sterile 10ml glass vials. The air in the headspace was purged with nitrogen gas and the glass vials were closed under aseptic conditions with sterile teflon ^{™} coated rubber bungs and sealed using flip-off seals.

### Example XI:

Ifosfamide (10g) and HPBCD (80g), Disodium hydrogen phosphate (0.1 g in 10ml of water) and Sodium hydrogen phosphate (0.06g in 10ml of water) were taken in a graduated flask. Water for injection was slowly added into it with intermittent mixing to get 200ml clear homogenous solution. The resultant solution was passed through sterile 0.2µm filter and filled aseptically in sterile 10ml glass vials. The air in the headspace was purged with nitrogen gas and the glass vials were closed under aseptic conditions with sterile teflon ^{™} coated rubber bungs and sealed using flip-off seals.

### Example XII:

HPBCD (20g) was dissolved in 40 ml of water. To the concentrated solution of HPBCD, Ifosfamide(10g) was added gradually and the mixture was stirred at a moderate speed for 1 hour. The clear solution was then diluted to 200ml with water. The resultant solution was filtered through 0.2µm filter and filled aseptically in sterile 10ml glass vials. The air in the headspace of the vials was purged with nitrogen gas and the glass vials were closed under aseptic conditions with sterile Teflon™ coated rubber bungs and sealed using flip off seals.

### Example XIII:

The composition was prepared by following the procedure of Example IV using the components in the amounts mentioned in table no 8.

### Example XIV:

HPBCD(40g) was dissolved in 80 ml of water. To the concentrated solution of HPBCD, Ifosfamide(100g) was added gradually and dissolved by stirring. The volume was made up to 200ml with water and mixed. The resultant solution was filtered through 0.2µm filter and filled aseptically in sterile 10 ml glass vials. The air in the headspace of the vials was purged with nitrogen gas and the glass vials were closed under aseptic conditions with sterile Teflon™ coated rubber bungs and sealed using flip off seals. The composition of this Example was analysed for Ifosfamide content by HPLC and was found to contain 500.3mg/ml of Ifosfamide.

### Example XV:

The composition was prepared by following the procedure of Example XIV using the components in the amounts mentioned in table no 8. The composition of this Example was analysed for Ifosfamide content by HPLC and was found to contain 500.28mg/ml of Ifosfamide.

### Example XVI:

Ifosfamide (200g) and HPBCD (10g) were taken in a in a graduated flask. Water for injection was slowly added into it with intermittent mixing to get 200ml clear homogenous solution. The resultant solution was passed through sterile 0.2µm filter and filled aseptically in sterile 10ml glass vials. The air in the headspace was purged with nitrogen gas and the glass vials were closed under aseptic conditions with sterile teflon^{™} coated rubber bungs and sealed using flip-off seals. The composition of this Example was analysed for Ifosfamide content by HPLC and was found to contain 1025.5mg/ml of Ifosfamide.

## Claims

1. Stable, clear, aqueous Ifosfamide composition having reduced toxicity, for parenteral administration comprising
Ifosfamide up to 1100 mg /ml of the composition
and 2-hydroxypropyl-β-cyclodextrin, with the proviso that the composition does not contain sodium 2-mercaptoethanesulphonate.

2. Ifosfamide composition as claimed in claim 1, wherein the molar substitution by hydroxy propyl groups in 2-hydroxypropyl-β-cyclodextrin is 0.05 - 2, preferably 0.3 - 1.5, and more preferably 0.5 -1.2.

3. Ifosfamide composition as claimed in claim 1 or 2, wherein the molar ratio of Ifosfamide to 2-hydroxypropyl-β-cyclodextrin is 100:0.1 - 1:300, preferably 100:0.25 - 1: 100, more preferably 100:1 - 1: 20, and most preferably 100:3.3 - 1:2.5.

4. Ifosfamide composition as claimed in claim 1, further comprising pharmaceutically acceptable additives, wherein, preferably, said additives are selected from buffers, tonicity agents, preservatives, chelating agents, antioxidants and anticrystallising agents.

5. Ifosfamide composition as claimed in any of claims 1-4, wherein the Ifosfamide content of the composition is 1 - 200 mg/ml, or 10 - 100 mg/ml, or 40 - 50 mg/ml, or 200- 500 mg/ml, or 500-1000 mg/ml.

6. Ifosfamide composition as claimed in any of claims 1-5, wherein the pH of the composition is between 3.0 - 9.0, preferably 5:0 -8.0.

7. Ifosfamide composition as claimed in claim 4, wherein the buffering agent is selected from sodium dihydrogen phosphate, disodium hydrogen phosphate, dipotassium hydrogen phosphate, potassium dihydrogen phosphate, phosphoric acid, histidine hydrochloride, sodium hydroxide, hydrochloric acid and mixtures thereof, wherein, preferably, the buffering agent is a mixture of sodium dihydrogen phosphate and disodium hydrogen phosphate.

8. Ifosfamide composition as claimed in claim 1, wherein each millilitre of the composition comprises
Ifosfamide: 50 mg,
2-hydroxypropyl-β-cyclodextrin : 100mg,
sodium dihydrogen phosphate: 0.3mg and
disodium hydrogen phosphate 0.5mg; or
wherein each millilitre of the composition comprises
Ifosfamide: 500 mg
and 2-hydroxypropyl-β-cyclodextrin: 400mg; or
wherein each millilitre of the composition comprises
Ifosfamide: 1000 mg
and 2-hydroxypropyl-β-cyclodextrin : 50mg.

9. A process for the preparation of an Ifosfamide composition as claimed in any claims 1-8 comprising the steps of
i. bringing in intimate contact Ifosfamide, 2-hydroxypropyl-β-cyclodextrin and water; and
ii. rendering sterile the composition so obtained at the end of step (i).

10. A process as claimed in claim 9 further comprising addition of required quantities of pharmaceutically acceptable additives while bringing in intimate contact Ifosfamide, 2-hydroxypropyl-β-cyclodextrin and water.

11. A process as claimed in claim 9 or 10, wherein the composition is rendered sterile by passing through a sterile 0.2µm sterilizing grade filter.

12. A process as claimed in any of claims 9-11, further comprising the steps of transferring aseptically the sterile composition into a sterile container, purging the air in the headspace of the container with inert gas and sealing the container.

## Patentansprüche

1. Stabile, klare, wässrige Ifosfamid-Zusammensetzung mit verminderter Toxizität zur parenteralen Verabreichung, umfassend:
Ifosfamid bis zu 1100 mg/ml der Zusammensetzung
und 2-Hydroxypropyl-β-cyclodextrin, mit der Maßgabe, dass die Zusammensetzung kein Natrium-2-mercaptoethansulfonat enthält.

2. Ifosfamid-Zusammensetzung nach Anspruch 1, worin die molare Substitution durch Hydroxypropylgruppen in 2-Hydroxypropyl-β-cyclodextrin 0,05 bis 2, bevorzugt 0,3 bis 1,5 und bevorzugter 0,5 bis 1,2 beträgt.

3. Ifosfamid-Zusammensetzung nach Anspruch 1 oder 2, worin das Molverhältnis von Ifosfamid zu 2-Hydroxypropyl-β-cyclodextrin bei 100:0,1 bis 1:300, bevorzugt 100:0,25 bis 1:100, bevorzugter 100:1 bis 1:20 und am bevorzugtesten 100:3,3 bis 1:2,5 liegt.

4. Ifosfamid-Zusammensetzung nach Anspruch 1, die ferner pharmazeutisch verträgliche Hilfsstoffe umfasst, worin die Hilfsstoffe bevorzugt aus Puffern, Tonizitätsmitteln, Konservierungsmitteln, Chelatbildnern, Antioxidanzien und Antikristallisationsmitteln ausgewählt sind.

5. Ifosfamid-Zusammensetzung nach einem der Ansprüche 1 bis 4, worin der Ifosfamidgehalt der Zusammensetzung 1 bis 200 mg/ml oder 10 bis 100 mg/ml oder 40 bis 50 mg/ml oder 200 bis 500 mg/ml oder 500 bis 1000 mg/ml beträgt.

6. Ifosfamid-Zusammensetzung nach einem der Ansprüche 1 bis 5, worin der pH der Zusammensetzung zwischen 3,0 bis 9,0, bevorzugt 5,0 bis 8,0 liegt.

7. Ifosfamid-Zusammensetzung nach Anspruch 4, worin das Puffermittel aus Natriumdihydrogenphosphat, Dinatriumhydrogenphosphat, Dikaliumhydrogenphosphat, Kaliumdihydrogenphosphat, Phosphorsäure, Histidinhydrochlorid, Natriumhydroxid, Salzsäure und Gemischen davon ausgewählt ist, worin das Puffermittel bevorzugt ein Gemisch von Natriumdihydrogenphosphat und Dinatriumhydrogenphosphat darstellt.

8. Ifosfamid-Zusammensetzung nach Anspruch 1, worin jeder Milliliter der Zusammensetzung Folgendes umfasst:
Ifosfamid: 50 mg,
2-Hydroxypropyl-β-cyclodextrin: 100 mg,
Natriumdihydrogenphosphat: 0,3 mg und
Dinatriumhydrogenphosphat: 0,5 mg; oder
worin jeder Milliliter der Zusammensetzung Folgendes umfasst:
Ifosfamid: 500 mg
und 2-Hydroxypropyl-β-cyclodextrin: 400 mg; oder
worin jeder Milliliter der Zusammensetzung Folgendes umfasst:
Ifosfamid: 1000 mg
und 2-Hydroxypropyl-β-cyclodextrin: 50 mg.

9. Verfahren zur Herstellung einer Ifosfamidzusammensetzung nach einem der Ansprüche 1 bis 8, das die folgenden Schritte umfasst:
i. in innigen Kontakt bringen von Ifosfamid, 2-Hydroxypropyl-β-cyclodextrin und Wasser; und
ii. Sterilisieren der auf diese Weise erhaltenen Zusammensetzung am Ende von Schritt (i).

10. Verfahren nach Anspruch 9, das ferner das Zufügen von erforderlichen Mengen von pharmazeutisch verträglichen Hilfsstoffen umfasst, während Ifosfamid, 2-Hydroxypropyl-β-cyclodextrin und Wasser in innigen Kontakt miteinander gebracht werden.

11. Verfahren nach Anspruch 9 oder 10, worin die Zusammensetzung mittels Durchgeben durch ein steriles 0,2-µm-Filter von Sterilisierungsqualität sterilisiert wird.

12. Verfahren nach einem der Ansprüche 9 bis 11, das ferner die Schritte des aseptischen Überführens der sterilen Zusammensetzung in ein steriles Behältnis, des Spülens der Luft im Kopfraum des Behältnisses mit inertem Gas und des festen Verschließens des Behältnisses umfasst.

## Revendications

1. Composition d'iphosphamide aqueuse, limpide, stable présentant une toxicité réduite, destinée à une administration par voie parentérale, comprenant
de l'iphosphamide jusqu'à 1100 mg/ml de la composition
et de la 2-hydroxypropyl-β-cyclodextrine, à condition que la composition ne contienne pas de 2-mercaptoéthanesulfonate de sodium.

2. Composition d'iphosphamide selon la revendication 1, dans laquelle la substitution molaire par des groupements hydroxypropyle dans 2-hydroxypropyl-β-cyclodextrine est de 0,05 - 2, de préférence de 0,3 - 1,5 et plus préférablement de 0,5 -1,2.

3. Composition d'iphosphamide selon la revendication 1 ou 2, dans laquelle le rapport molaire de l'iphosphamide à la 2-hydroxypropyl-β-cyclodextrine est de 100:0,1 -1:300, de préférence de 100:0,25 - 1:100, plus préférablement de 100:1 - 1:20 et le plus préférablement de 100:3,3 - 1:2,5.

4. Composition d'iphosphamide selon la revendication 1, comprenant en outre des additifs pharmaceutiquement acceptables, où lesdits additifs sont choisis de préférence parmi des tampons, des agents de tonicité, des conservateurs, des chélateurs, des antioxydants et des agents anti-cristallisation.

5. Composition d'iphosphamide selon l'une quelconque des revendications 1 à 4, dans laquelle la teneur en iphosphamide de la composition est de 1 - 200 mg/ml ou de 10 - 100 mg/ml ou de 40 - 50 mg/ml ou de 200 - 500 mg/ml ou de 500 - 1000 mg/ml.

6. Composition d'iphosphamide selon l'une quelconque des revendications 1 à 5, dans laquelle le pH de la composition est compris entre 3,0 - 9,0, de préférence 5,0 - 8,0.

7. Composition d'iphosphamide selon la revendication 4, dans laquelle le tampon est choisi parmi le dihydrogénophosphate de sodium, l'hydrogénophosphate disodique, l'hydrogénophosphate de dipotassium, le dihydrogénophosphate de potassium, l'acide phosphorique, le chlorhydrate d'histidine, l'hydroxyde de sodium, l'acide chlorhydrique et des mélanges de ceux-ci, où, de préférence, le tampon est un mélange de dihydrogénophosphate de sodium et l'hydrogénophosphate disodique.

8. Composition d'iphosphamide selon la revendication 1, dans laquelle chaque millilitre de la composition comprend
iphosphamide : 50 mg,
2-hydroxypropyl-β-cyclodextrine : 100 mg,
dihydrogénophosphate de sodium : 0,3 mg et
hydrogénophosphate disodique : 0,5 mg ; ou
dans laquelle chaque millilitre de la composition comprend
iphosphamide : 500 mg,
et 2-hydroxypropyl-β-cyclodextrine : 400 mg ; ou
dans laquelle chaque millilitre de la composition comprend
iphosphamide : 1000 mg,
et 2-hydroxypropyl-β-cyclodextrine : 50 mg.

9. Procédé de préparation d'une composition d'iphosphamide selon l'une quelconque des revendications 1 à 8, comprenant les étapes consistant à
i. amener en contact intime l'iphosphamide, la 2-hydroxypropyl-β-cyclodextrine et de l'eau ; et
i. rendre stérile la composition ainsi obtenue à la fin de l'étape (i).

10. Procédé selon la revendication 9, comprenant en outre l'addition de quantités requises d'additifs pharmaceutiquement acceptables au moment d'amener en contact intime l'iphosphamide, la 2-hydroxypropyl-β-cyclodextrine et l'eau.

11. Procédé selon la revendication 9 ou 10, dans lequel la composition est rendue stérile par le passage à travers un filtre stérile de qualité stérilisation de 0,2 µm.

12. Procédé selon l'une quelconque des revendications 9 à 11, comprenant en outre les étapes consistant à transférer de manière aseptique la composition stérile dans un conteneur stérile, purger l'air dans la partie haute du conteneur au moyen de gaz inerte et sceller le conteneur.
